# FASCICULE DE BREVET EUROPEEN

(11) **EP 0 603 920 B1**
(45) Date de publication et mention de la délivrance du brevet: **24.03.1999**
(21) Numéro de dépôt: 93203267.5
(22) Date de dépôt: 23.11.1993
(51) Int. Cl.: A61L 27/00

(54) **Procédé de traitement de tissus osseux et biomateriaux implantables correspondants**
Verfahren zur Behandlung von Knochengeweben und implantierbare Biomaterialien dafür
Method for the treatment of bone tissues and implantable biomaterials thereof

(30) Priorité: 21.12.1992 FR 9215577
(43) Date de publication de la demande: 29.06.1994
(73) Titulaire: BIOLAND Société à Responsabilité Limitée, F-31100 Toulouse (FR)
(72) Inventeur: Fages, Jacques, F-31120 Portet sur Garonne (FR); Marty, Alain, F-31400 Toulouse (FR); Combes, Didier, F-31750 Escalquens (FR); Condoret, Jean-Stéphane, F-31400 Toulouse (FR)
(74) Mandataire: Cabinet BARRE LAFORGUE & associés

(56) Documents cités:
- WO-A-87/03812
- WO-A-91/07194
- GB-A- 2 175 506

## Description

La présente invention concerne un procédé de traitement de tissus osseux et des biomatériaux implantables correspondants.

Plus particulièrement, un tel procédé permet de traiter un tissu osseux en vue de son implantation chez l'homme. Un tel tissu ne doit pas provoquer de rejet (ou du moins le moins possible), et doit présenter une bonne capacité ostéoconductrice, c'est-à-dire, qu'il doit autoriser la formation et la migration de tissu osseux néoformé par le receveur.

La greffe de tissu osseux est une technique utilisée quotidiennement dans la plupart des services de chirurgie orthopédique à travers le monde. Ces greffes peuvent être de trois types à savoir :
- les allogreffes,
- les autogreffes,
- les xénogreffes.

Les allogreffes osseuses consistent à transplanter du tissu osseux d'un donneur vers un receveur de même espèce, mais différent du donneur. Les autogreffes osseuses consistent à prélever du tissu osseux et à le greffer sur un même individu. Les xénogreffes osseuses consistent à transplanter du tissu osseux d'un animal (souvent porcin ou bovin) vers un individu humain.

Pour réaliser ces greffes, on part d'un tissu osseux que l'on traite pour le nettoyer mécaniquement et l'épurer de toutes les substances qui nuiraient a son implantation. Ce faisant, on constate, avec les traitements utilisés dans l'art antérieur, que les propriétés mécaniques du tissu osseux sont altérées. En effet, les substances organiques extraites participent aux propriétés mécaniques de l'os. Or, la greffe d'un implant osseux est en général réalisée à des fins de restructuration du squelette, dans une portion soumise à des problèmes de résistance où les propriétés mécaniques sont primordiales. En conséquence, il serait avantageux de disposer de greffons osseux ayant des propriétés mécaniques équivalentes à celles de l'os naturel, voire supérieures.

Par exemple, de tels greffons seraient indiqués dans des applications orthopédiques et en particulier lorsque le greffon est mis en charge, à savoir notamment : chirurgie du rachis (fusion cervicale, remplacement des disques lombaires, ...), reconstruction du fond de cotyle, chirurgie de reprise d'arthroplastie, ostéotomies, pseudarthroses, arthrodèses, ...

Ainsi, lorsque le greffon est utilisé pour restaurer une partie de squelette supportant une prothèse artificielle implantée (prothèse d'articulation, prothèse dentaire...), l'utilisation d'un greffon dont la résistance mécanique, notamment en compression, serait supérieure à celle de l'os d'origine qui s'est détérioré, constituerait un avantage déterminant du point de vue de la longévité de fonctionnement de la prothèse. Et, le greffon interposé entre la prothèse et l'os naturel rendrait les variations de propriétés mécaniques plus progressives et améliorerait la qualité de la transmission des contraintes en évitant des gradients localisés trop importants de résistance. Egalement, si le greffon a, dès son implantation, des résistances équivalentes à celles de l'os, on supprime tout risque de détérioration pendant la résorption du greffon par un tissu osseux néoformé.

L'invention vise donc à proposer un biomatériau implantable dont les propriétés mécaniques, notamment la résistance en compression, sont au moins équivalentes à celles de l'os naturel. Plus particulièrement, l'invention vise à proposer un biomatériau implantable dont la résistance en compression peut être comprise entre 1 et 2 fois celle de l'os naturel.

Par ailleurs, l'invention vise aussi à proposer un biomatériau qui améliore l'efficacité des greffes osseuses, tant du point de vue mécanique que du point de vue biologique.

En effet, les allogreffes présentent de nombreux inconvénients. Tout d'abord, les risques infectieux liés à la transmission du tissu osseux d'un individu vers un autre sont nombreux. Tel est le cas, notamment, en ce qui concerne la transmission du virus HIV du SIDA. Etant donné le développement important de cette maladie, un tel risque infectieux s'est considérablement accru ces dernières années. Outre ces risques infectieux, qui concernent également d'autres virus, les principales complications liées à l'utilisation d'allogreffes sont des fractures, une mauvaise recolonisation des tissus osseux implantés (greffons) et des rejets de l'implant. La mauvaise recolonisation des greffons pose aujourd'hui un problème important. En effet, les tissus du greffon sont sensés être résorbés, envahis puis remplacés par un tissu osseux néoformé. Mais cette réhabilitation est jusqu'à présent plutôt faible.

Un des buts de la présente invention est donc aussi de créer un tissu osseux implantable sûr au niveau infectieux et immunitaire.

Un autre but de l'invention est qu'un tel tissu osseux présente une bonne capacité ostéoconductrice (c'est-à-dire facilite une bonne recolonisation des greffons).

Les autogreffes sont souvent préférées aux allogreffes car elles sont mieux recolonisées et susceptibles d'apporter des cellules osseuses sur le site de la greffe. Il en résulte que les risques infectieux et immunitaires sont notablement réduits, mais ce type de pratique n'est pas totalement satisfaisante. En effet, elle est douloureuse, souvent mal acceptée par le patient, et présente des risques de complication au niveau du site donneur. En outre, de nombreuses interventions requièrent de grandes quantités de tissu osseux incompatibles avec l'autogreffe.

Les xénogreffes osseuses présentent elles aussi de nombreux inconvénients. En général, ces greffes provoquent de fortes réactions immunitaires (rejet). Pour pallier cet inconvénient, diverses tentatives visant à atténuer ou supprimer ces réactions ont été effectuées. Leur principe réside généralement en une déprotéinisation du tissu osseux avant implant. En effet, les protéines contenues dans le tissu osseux sont à l'origine d'une partie des réactions de rejet. Ces réactions de rejet sont également liées à la présence de débris cellulaires dans les tissus médullaires et aux autres éléments de ces tissus.

Pour extraire les protéines du tissu osseux, il est connu d'utiliser des solvants organiques. Les solvants les plus utilisés sont : l'éthylène diamine, le peroxyde d'hydrogène, divers solvants chlorés tels le chloroforme ou le dichlorométhane, mais aussi l'éthanol et l'acétone. La plupart des tissus osseux ainsi déprotéinés ne présentent pas ou peu de réactions immunitaires. Ils sont généralement bien revascularisés et sont envahis par des cellules ostéogéniques du receveur. Cependant ils ne présentent pas eux-mêmes de propriétés ostéoinductrices. Leur résistance mécanique est généralement inférieure à celle de l'os naturel ou du même ordre.

On remarquera cependant que les solvants utilisés pour la déprotéinisation sont souvent hautement toxiques. De ce fait, les tissus osseux doivent être soigneusement rincés (ce qui n'est pas facile, étant donné leur porosité) pour éviter toute pollution du site receveur.

Le brevet FR-A-2.654.625 décrit un procédé de déprotéinisation utilisant de tels solvants toxiques associés à un agent d'extraction sélectif à base d'urée. Un tel procédé se solde malgré tout par une délipidation très hétérogène, puisque selon ce brevet il peut rester de 0,5 à 5 % de lipides.

Les fluides à l'état supercritique sont déjà connus pour extraire des matières diverses telles que lipides, protéines, nucléotides, saccharides de tissus ou organes d'animaux. Néanmoins, ces procédés connus sont considérés comme complexes et onéreux à mettre en oeuvre, sans fournir d'avantages prépondérants par rapport aux autres procédés d'extraction.

Ainsi, l'art antérieur n'enseigne pas la possibilité d'effectuer une extraction de matières organiques lors du traitement d'un tissu osseux à la suite de laquelle la résistance mécanique du tissu osseux est améliorée.

La présente invention a pour but de pallier l'ensemble des inconvénients de l'art antérieur et notamment de proposer un tissu osseux implantable de résistance améliorée par rapport à celle de l'os naturel, qui soit sûr au niveau infectieux et immunitaire, qui présente une bonne capacité ostéoconductrice et qui ne mette pas en oeuvre de produits toxiques. La présente invention a notamment pour but de proposer un procédé permettant le traitement d'un tel tissu osseux.

A cet effet, la présente invention concerne un procédé de traitement de tissus osseux animaux ou humains pour l'obtention d'un biomatériau implantable sur un être humain et destiné à subir des contraintes mécaniques, dans lequel on nettoie mécaniquement le tissu osseux de toutes les matières organiques qui l'entourent et on extrait du tissu osseux les matières organiques, caractérisé en ce qu'il comporte au moins une étape dans laquelle on fait pénétrer un fluide à l'état supercritique dans la totalité du tissu osseux.

Les inventeurs ont ainsi constaté avec surprise que le seul fait de placer le tissu osseux au contact d'un fluide supercritique au cours du traitement renforce sa résistance mécanique alors même que l'on extrait les matières organiques.

Selon l'invention, on fait pénétrer le fluide à l'état supercritique dans le tissu osseux lors d'une étape d'extraction des matières organiques essentiellement lipidiques présentes dans le tissu et qui sont solubilisées dans le fluide à l'état supercritique. L'extraction ainsi réalisée par ce fluide supercritique présente des qualités qui se trouvent particulièrement appropriées au tissu osseux.

Selon l'invention, le procédé consiste à :
a) nettoyer mécaniquement le tissu osseux de toutes les matières organiques qui l'entourent,
b) découper ce tissu osseux selon une forme prédéterminée,
c) nettoyer le tissu osseux pour en extraire des constituants nocifs à une bonne réimplantation, en faisant pénétrer dans la totalité du tissu osseux, un fluide à l'état supercritique adapté pour solubiliser et extraire les matières organiques essentiellement lipidiques présentes dans ce tissu,
d) laver, déshydrater et stériliser le tissu osseux ainsi nettoyé. L'étape de nettoyage c) consiste en outre à traiter de manière chimique et/ou enzymatique le tissu osseux pour en extraire des protéines spécifiques restantes.

Le fluide à l'état supercritique c est-à-dire présentant une viscosité dynamique faible (proche de celle d'un gaz), un coefficient de diffusion élevé, une très faible tension interfaciale et une masse volumique élevée (proche de celle d'un liquide) diffuse facilement à travers le matériau poreux sans aucun problème de mouillabilité. En outre, le pouvoir solvant d'un tel fluide est élevé (proche de celui des liquides et pouvant atteindre 10⁸ fois celui d'un gaz), et peut être modulé en faisant varier la pression. Il en résulte qu'un tel fluide à l'état supercritique dissous facilement et pratiquement totalement les matières organiques essentiellement lipidiques présentes dans le tissu osseux. Les risques immunitaires et infectieux s'en trouvent considérablement amoindris.

Un des avantages de l'invention réside dans l'utilisation du dioxyde de carbone (CO₂) en tant que fluide à l'état supercritique, en effet, ce composant présente de nombreuses propriétés intéressantes, à savoir :
- sa température critique, de 31° C, est basse. On peut ainsi avoir du dioxyde de carbone a l'état supercritique en travaillant à une température de l'ordre de 31° pour une pression de l'ordre de 7,38 MPa. Selon la pression appliquée, on peut donc travailler à des températures comprises entre 31° et 60° C auxquelles la seule altération du tissu osseux possible est une dénaturation du collagène qui lui enlève son caractère antigénique,
- son pouvoir solvant est excellent, en particulier pour les lipides. Il est par exemple connu que de nombreux acides gras et triglycérides peuvent avoir des solubilités dans le dioxyde de carbone à l'état supercritique pouvant aller jusqu'à 10%. Or ces lipides sont présents en grande quantité dans les tissus médullaires qui imprègnent l'os spongieux, ils sont fortement antigéniques et toujours très difficiles à éliminer ; de plus n'étant pas mouillables, ils constituent une barrière physique prévenant la recolonisation du greffon,
- il renforce les propriétés biomécaniques de l'os et favorise l'ostéointégration du tissu osseux implanté,
- c'est un produit naturel dépourvu de toute toxicité,
- il est facile à se procurer et ne présente pas de danger ni pour le produit traité, ni pour l'expérimentateur.

Avantageusement, le traitement du tissu osseux est complété par un traitement chimique ou enzymatique plus particulièrement adapté pour extraire les matières protéiques présentes dans le tissu osseux.

La présente invention concerne également un biomatériau implantable obtenu selon le procédé ci-dessus indiqué, et constitué par un tissu osseux épuré et ayant été placé au contact d'un fluide à l'état supercritique, ledit tissu étant adapté pour être implanté chez l'homme au niveau d'un tissu osseux lésé.

Selon l'invention, le tissu osseux a été épuré des matières organiques essentiellement lipidiques par extraction à l'aide du fluide à l'état supercritique.

Un tel matériau présente une résistance équivalente ou supérieure à celle de l'os naturel, est sûr au niveau infectieux et immunitaire, ne présente pas de toxicité, et du fait de l'extraction quasi complète des lipides qu'il contenait à l'origine, offre une bonne capacité ostéoconductrice.

D'autres objets, caractéristiques et avantages de la présente invention, ressortiront de la description qui va suivre, à titre d'exemple non limitatif, qui se réfère au schéma annexé représentant une installation pour la mise en oeuvre du procédé selon l'invention.

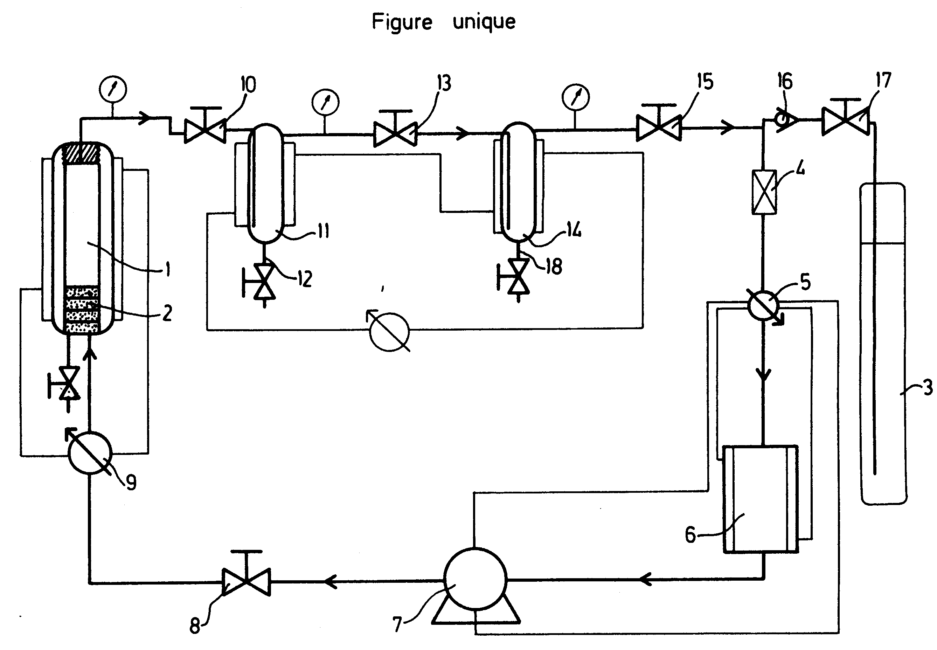

L'installation représentée sur la figure comporte un réacteur 1 dans lequel on place le tissu osseux 2 et à travers lequel on entretient un courant de dioxyde de carbone CO₂ à l'état supercritique.

Le dioxyde de carbone est stocké à l'état liquide dans une bouteille 3, traverse un filtre 4, est refroidi par un groupe réfrigérant 5 à une température où il est certainement liquide, de l'ordre de 0° C, puis introduit dans un réservoir tampon 6. Une pompe 7 doseuse est placée en aval du réservoir 6 pour comprimer le dioxyde de carbone liquide à une pression permettant son passage à l'état supercritique. Une vanne 8 est placée en aval de la pompe 7 pour isoler le réacteur 1 en vue de son chargement ou de son déchargement. Avant son introduction dans le réacteur 1, le dioxyde de carbone est réchauffé par un réchauffeur 9, de sorte qu'à la sortie de ce réchauffeur 9, le dioxyde de carbone se trouve à l'état supercritique. Le dioxyde de carbone traverse le réacteur 1 et en sort à une extrémité opposée à son extrémité d'introduction. Une vanne 10 est placée en aval du réacteur 1 pour permettre de l'isoler du circuit. La vanne 10 permet également de réaliser une chute de pression avant l'introduction du dioxyde de carbone dans un premier séparateur 11. Les matières dissoutes dans le dioxyde de carbone sont récupérées à la sortie 12 du séparateur 11. L'installation comprend aussi un deuxième étage de séparation comprenant une vanne 13 faisant encore chuter la pression et un deuxième séparateur 14 ayant une sortie 18 de récupération des matières organiques. On peut prévoir plus de deux étages de séparation, selon les besoins. Le dioxyde de carbone, à la sortie 12, 18 des étages de séparation, est recyclé dans le circuit par l'intermédiaire d'une vanne 15. La bouteille 3 est reliée à ce circuit fermé par l'intermédiaire d'un clapet anti-retour 16 et d'une vanne 17.

Le procédé de traitement de tissu osseux selon l'invention est décrit ci-après.

De manière classique, on procède en premier lieu à un nettoyage mécanique de l'os, puis à sa découpe aux dimensions souhaitées et selon une forme prédéterminée. La forme du tissu osseux peut par exemple être un parallélépipède rectangle, un cylindre ou tout autre forme appropriée. Ce tissu osseux est alors placé dans le réacteur 1 d'extraction. Les conditions opératoires optimales sont réunies pour une température comprise entre 31° et 60° C et une pression de l'ordre de 1,5.10⁷ à 4.10⁷ Pa.

Le dioxyde de carbone CO₂, est pompé sous forme liquide par la pompe 7 doseuse. Ce liquide est préchauffé en amont du réacteur 1 d'extraction afin d'être introduit dans celui-ci à l'état supercritique.

On rappelle que les fluides à l'état supercritique peuvent être définis comme des gaz placés dans des conditions de température et de pression telles que leurs propriétés sont intermédiaires entre celles des gaz et celles des liquides. On les nomme encore "gaz denses" ou "liquide expansé". Pour un corps chimique donné, le point précis du diagramme température-pression pour lequel les deux phases liquide et vapeur n'en forment plus qu'une est appelé point critique. Au-delà de cette température critique (Tc) et de cette pression critique (Pc), le fluide est en l'état dit "supercritique".

En traversant le réacteur 1, le dioxyde de carbone à l'état supercritique solubilise une grande partie des matières organiques, essentiellement lipidiques de l'os. En particulier, il dissout les graisses des tissus médullaires contenues dans le tissu osseux.

En raison des propriétés des fluides supercritiques, cette extraction est très efficace. Notamment, les graisses normalement inaccessibles aux solvants chimiques utilisés dans les procédés connus sont extraites avec le dioxyde de carbone à l'état supercritique.

Pour contrôler l'extraction lipidique réalisée par le dioxyde de carbone, on vide en permanence les séparateurs 11 et 14 de l'extrait en récupérant les résidus gras. On laisse le tissu osseux au contact du dioxyde de carbone supercritique pendant qu'un débit de résidus gras est récupéré à la sortie 12, 18 des séparateurs 11, 14. La durée du traitement varie en fonction du poids de tissu osseux à traiter et du débit de dioxyde de carbone supercritique introduit dans le réacteur 1. On peut ainsi interrompre le procédé en contrôlant la masse de dioxyde de carbone traversant le tissu osseux dont la masse est elle-même déterminée. On sait en effet que le rendement massique de l'extraction est une constante. Ainsi, à l'aide d'un débitmètre massique placé en série à la sortie du réacteur 1, on peut interrompre le procédé lorsque la masse de dioxyde de carbone utilisé est suffisante.

On notera que les éléments extraits se composent à plus de 98 % de graisse. Le tissu osseux quant à lui, contient après traitement en moyenne moins de 2 % de graisse, et ceci de manière homogène. Et sa résistance à la compression est de l'ordre de 10 à 20 MPa.

On notera que, contrairement à ce qui se passe de manière classique dans un réacteur chimique, ce n'est pas l'extrait (matière essentiellement lipidique) qu'on utilise par la suite, mais le reliquat (le tissu osseux).

Et le fluide à l'état supercritique présente ainsi la double fonction de renforcer le matériau osseux, ce qui améliore ses propriétés biomécaniques, et d'épurer le matériau osseux.

Le tissu osseux ainsi traité subit une action complémentaire classique de traitement chimique ou enzymatique pour extraire des protéines spécifiques. Le traitement complémentaire chimique peut être réalisé à partir de peroxyde d'hydrogène, tandis que le traitement enzymatique peut être effectué à l'aide de protéase. Ce traitement complémentaire assure une meilleure extraction des protéines du tissu osseux et diminue d'autant le risque de rejet du tissu osseux ainsi traité.

Ensuite, le tissu osseux est soumis à un lavage. Ce lavage est réalisé dans plusieurs bains successifs d'eau distillée à une température comprise entre 30° et 60° C.

Une étape de déshydratation et de désinfection du tissu osseux est ensuite réalisée. Cette étape est réalisée par passage dans plusieurs bains successifs d'éthanol de concentration croissante, par exemple 70, 95 et 100 %. On notera que l'éthanol étant un excellent virucide, il permet simultanément de déshydrater le tissu et d'accroître la sécurité infectieuse du biomatériau. Un séchage en étuve ventilée à une température comprise entre 30 et 60° C complète ce procédé.

Le tissu osseux est alors soumis à une stérilisation après avoir été emballé. Cette stérilisation peut être effectuée par irradiation soit aux particules bêta, soit aux rayons gamma (25 k Gray). Une éventuelle nouvelle taille du tissu osseux avant implant est possible pour l'adapter au tissu osseux receveur. En effet, le tissu osseux ainsi traité est relativement dur et peut se retailler sans s'effriter.

Dans le tableau ci-après, on trouvera les résultats d'analyses d'un tissu osseux non traité (exemple 1), d'un tissu osseux traité uniquement à l'aide du CO₂ à l'état supercritique (exemple 2), et de tissus osseux traités au CO₂ et ayant subi une étape complémentaire, soit chimique (exemple 3), soit enzymatique (exemple 4).

Les valeurs respectives de teneur en matière organique MO, en carbone organique C, en azote N, en résidu lipidique (lipides) et la résistance à la compression sont données pour chacun de ces quatre exemples. Les valeurs indiquées sont des valeurs moyennes ; entre parenthèses est donné l'intervalle de tolérance.

Les tissus osseux analysés proviennent d'extrémités distales de fémur de bovin.

| | Exemple 1 : os non traité | Exemple 2 : os traité au CO₂ | Exemple 3 : os traité au CO₂ + peroxyde d'hydrogène H₂O₂ | Exemple 4 : os traité au CO₂ + protéase |
|---|---|---|---|---|
| MO (%) | 62,7 (50,0-70,3) | 23,0 (21,6-25,7) | 20,8 (18,9-21,9) | 20,6 (16,1-23,6) |
| C (%) | 44,8 (43,1-46,5) | 10,8 (10,1-12,4) | 9,2 (8,7-10,3) | 9,8 (8,8-11,6) |
| N (%) | 2,4 (2,2-2,6) | 4,1 (3,9-4,3) | 3,9 (3,3-4,3) | 4,0 (3,0-4,7) |
| lipides (%) | 51,3 (24,7-77,3) | 1,9 (0,6-5,3) | 1,5 (0,8-3,0) | 1,7 (0,8-2,8) |
| Résistance à la compression | 8,9 MPa | 16,9 MPa | 10,7 MPa | 11,5 MPa |

Une deuxième série d'essais identiques a été réalisée à partir d'os bovins d'une autre origine. Dans le tableau ci-après, les résultats sont donnés en moyenne sur 38 mesures pour les exemples 1 et 4, 39 mesures pour l'exemple 2 et 28 mesures pour l'exemple 3.

| | Exemple 1 | Exemple 2 | Exemple 3 | Exemple 4 |
|---|---|---|---|---|
| MO (%) | 62,1 | 22,8 | 19,6 | 20,6 |
| C(%) | 46,6 | 10,8 | 8,6 | 9,6 |
| N(%) | 2,4 | 4,1 | 3,6 | 4,1 |
| lipides (%) | 51,0 | 1,1 | 0,7 | 1,3 |
| Résistance à la compression | 10.42 MPa | 14,91 MPa | 10,65 MPa | 11,54 MPa |

Par rapport au seul traitement avec un fluide a l'état supercritique (exemple 2), on constate, pour les exemples 3 et 4, une baisse des teneurs en carbone et en azote, et plus généralement en matière organique. Cette baisse correspond à l'élimination de la fraction non lipidique des tissus médullaires ainsi qu'à la solubilisation des protéines solubles du tissu osseux. Cette opération permet de réduire considérablement les réactions inflammatoires et améliore encore l'ostéoconduction comme l'ont montré des expériences effectuées sur l'animal.

On notera que les propriétés biomécaniques de ces implants sont statistiquement meilleures que celles de l'os, à partir duquel ils sont réalisés (exemple 1). En effet, la résistance à la compression d'un tissu osseux traité est supérieure à celle du tissu osseux témoin non traité.

On notera en variante du procédé ci-dessus décrit, que les étapes de nettoyage et de découpage du tissu osseux peuvent être effectuées de la manière suivante.

L'étape de nettoyage peut être effectuée en utilisant un procédé de sablage où du corindon (alumine) est envoyé sous une pression comprise entre 4.10⁵ et 10⁶ Pa sur l'os afin de le débarrasser de tous les tissus mous adhérents, y compris le périoste. Cette technique est beaucoup plus rapide et efficace que les méthodes traditionnelles qui sont toutes manuelles alors que le sablage peut être effectué dans des appareils robotisés entièrement automatiques.

L'étape de découpe peut, quant à elle, être une découpe par jet d'eau. Une telle découpe permet une précision pouvant aller jusqu'à 10µm, elle est effectuée par un jet d'eau pure évitant donc tout risque de pollution par l'outil de découpe et elle est industrialisable.

Bien entendu, la présente invention n'est pas limitée au mode de réalisation choisi et englobe toute variante à la portée de l'homme du métier. Ainsi, l'étape de nettoyage chimique réalisée à l'aide d'un fluide à l'état supercritique peut être complétée par l'ajout d'un fluide dissous dans le fluide à l'état supercritique, et dont l'action spécifique sur le tissu osseux pourra avoir lieu dans le réacteur même.

Il est alors possible de réaliser l'ensemble des réactions de déprotéinisation et de délipidation dans un même lieu (le réacteur) et en même temps. En outre, le fluide ainsi dissous dans le fluide à l'état supercritique se trouve à une température et à une pression extrêmement favorables pour ce type de réaction.

Ainsi, si on souhaite éliminer des protéines des tissus médullaires ou de la matrice extracellulaire osseuse, ou encore des débris cellulaires d'origine médullaire et dont le haut pouvoir antigénique est en grande partie responsable de réponses immunitaires, on peut avantageusement introduire dans le circuit du réacteur, des détergents, des oxydants de la matière organique, des enzymes spécifiques de telle ou telle réaction et plus généralement toute substance adéquate pour le traitement souhaité dans la mesure où elle est solubilisée par le CO₂ à l'état supercritique.

On notera que le procédé et le biomatériau ci-dessus décrits peuvent être réalisés à partir d'os humains ou animaux. Ils sont applicables à la réalisation d'un greffon, notamment pour des applications orthopédiques, et dans les applications où le greffon subit des contraintes mécaniques, par exemple : chirurgie du rachis (fusion cervicale, remplacement des disques lombaires, ...), reconstruction du fond de cotyle, chirurgie de reprise d'arthroplastie, ostéotomies, pseudarthroses, arthrodèses, ...

## Revendications

1. Procédé de traitement de tissus osseux animaux ou humains pour l'obtention d'un biomatériau implantable sur un être humain et apte à subir des contraintes mécaniques, dans lequel on nettoie mécaniquement le tissu osseux de toutes les matières organiques qui l'entourent et on extrait du tissu osseux les matières organiques, caractérisé en ce qu'il comporte au moins une étape dans laquelle on fait pénétrer un fluide a l'état supercritique dans la totalité du tissu osseux.

2. Procédé selon la revendication 1, caractérisé en ce que l'on place le tissu osseux dans un courant de fluide supercritique qui le traverse.

3. Procedé selon l'une des revendications précédentes, caractérisé en ce que ladite étape est une étape dans laquelle on extrait les matières organiques essentiellement lipidiques présentes dans le tissu qui sont solubilisées dans le fluide à l'état supercritique.

4. Procédé selon l'une des revendications précédentes, caractérisé en ce qu'il consiste à :
a) nettoyer mécaniquement le tissu osseux de toutes les matières organiques qui l'entourent,
b) découper ce tissu osseux selon une forme prédéterminée,
c) nettoyer le tissu osseux pour en extraire des constituants nocifs à une bonne réimplantation, en faisant pénétrer dans la totalité du tissu osseux, un fluide à l'état supercritique adapté pour solubiliser et extraire les matières organiques essentiellement lipidiques présentes dans ce tissu,
d) laver, déshydrater et stériliser le tissu osseux ainsi nettoyé.

5. Procédé selon la revendication 4, caractérisé en ce que l'étape de nettoyage c) consiste en outre à traiter de manière chimique et/ou enzymatique le tissu osseux pour en extraire des protéines spécifiques restantes.

6. Procédé selon l'une des revendications précédentes, caractérisé en ce que le fluide à l'état supercritique utilisé est du dioxyde de carbone CO₂.

7. Procédé selon la revendication 6, caractérisé en ce que le dioxyde de carbone CO₂ à l'état de fluide supercritique présente une température de l'ordre de 31° à 60° C, et est soumis à une pression de l'ordre de 1,5.10⁷ à 4.10⁷ Pa.

8. Procédé selon l'une des revendications précédentes, caractérisé en ce que le fluide à l'état supercritique contient des fluides spécifiques dissous, ces fluides spécifiques étant adaptés pour extraire du tissu osseux des protéines ou autres substances spécifiques.

9. Procédé selon l'une des revendications 1 à 8, caractérisé en que des fluides spécifiques adaptés pour extraire du tissu osseux des protéines spécifiques sont utilisés dans une étape de lavage effectuée après l'étape dans laquelle le fluide à l'état supercritique est utilisé.

10. Procédé selon l'une des revendications précédentes, caractérisé en ce qu'on nettoie mécaniquement le tissu osseux par sablage à l'alumine.

11. Procédé selon l'une des revendications précédentes, caractérisé en ce qu'on découpe le tissu osseux par jet d'eau sous haute pression.

12. Procédé selon l'une des revendications précédentes, caractérisé en ce qu'après le traitement du tissu osseux par le fluide a l'état supercritique, on le lave par passage dans plusieurs bains successifs d'eau distillée à une température comprise entre 20° et 60° C.

13. Procédé selon la revendication 12, caractérisé en ce qu'après avoir lavé le tissu osseux, on le déshydrate et on le désinfecte par passage dans plusieurs bains successifs d'éthanol de concentration croissante, puis par un séchage en étuve ventilée à une température comprise entre 31° et 60° C.

14. Procédé selon la revendication 13, caractérisé en ce qu'après avoir déshydraté le tissu osseux, on le stérilise après emballage par irradiation.

15. Biomatériau implantable sur un être humain susceptible d'être obtenu selon le procédé conforme à l'une des revendications précédentes, caractérisé en ce qu'il est constitué d'un tissu osseux animal ou humain épure et ayant été placé au contact d'un fluide à l'état supercritique, caractérisé en ce qu'il présente un résidu lipidique inférieur à 2 % et une résistance équivalente ou supérieure a celle de l'os naturel.

16. Biomatériau selon la revendication 15, caractérisé en ce qu'il présente une résistance à la compression de l'ordre de 10 à 20 MPa.

## Claims

1. Method for treating animal or human bone tissues to obtain a biomaterial which is implantable in a human being and is capable of withstanding mechanical stresses, wherein the bone tissue is cleaned from all organic materials surrounding it and the organic materials are extracted from the bone tissue, characterized in that it includes at least one step in which a fluid in the supercritical state is made to penetrate the entire bone tissue.

2. Method according to claim 1, characterized in that the bone tissue is placed in a stream of supercritical fluid which passes through it.

3. Method according to one of the preceding claims, characterized in that the said step is a step in which the essentially lipidic organic materials present in the tissue are extracted which are solubilized in the fluid in the supercritical state.

4. Method according to one of the preceding claims, characterized in that it consists of:
a) mechanically cleaning the bone tissue from all the organic materials which surround it,
b) cutting this bone tissue into a predetermined shape,
c) cleaning the bone tissue to extract the constituents that are harmful to successful reimplantation, by causing the entire bone tissue to be penetrated by a fluid in the supercritical state able to solubilize and extract the essentially lipidic organic materials present in this tissue,
d) washing, dehydrating and sterilizing the bone tissue cleaned in this manner.

5. Method according to claim 4, characterized in that the cleaning step c) additionally consists of treating the bone tissue in a chemical or enzymatic manner so as to extract therefrom specific remaining proteins.

6. Method according to one of the preceding claims, characterized in that the fluid used in the supercritical state is carbon dioxide CO₂.

7. Method according to claim 6, characterized in that the carbon dioxide CO₂ in the supercritical state has a temperature of the order of 31 to 60°C, and is subjected to a pressure of the order of 1.5 x 10⁷ to 4 x 10⁷ Pa.

8. Method according to one of the preceding claims, characterized in that the fluid in the supercritical state contains specific dissolved fluids, these specific fluids being able to extract proteins or other specific substances from the bone tissue.

9. Method according to one of claims 1 to 8, characterized in that the specific fluids able to extract specific proteins from the bone tissue are used in a washing step carried out after the step in which the fluid in the supercritical state is used.

10. Method according to one of the preceding claims, characterized in that the bone tissue is cleaned mechanically by sanding with alumina.

11. Method according to one of the preceding claims, characterized in that the bone tissue is cut up by means of a high pressure water jet.

12. Method according to one of the preceding claims, characterized in that after the bone tissue is treated with the fluid in the supercritical state, it is washed by passing it through several successive baths of distilled water at a temperature of between 20°C and 60°C.

13. Method according to claim 12, characterized in that after the bone tissue has been washed it is dehydrated and disinfected by passing it through several successive baths of ethanol at increasing concentrations, and by then drying it in a ventilated oven at a temperature of between 31°C and 60°C.

14. Method according to claim 13, characterized in that after the bone tissue has been dehydrated, it is sterilized by irradiation after it is packaged.

15. Biomaterial implantable in a human being which can be obtained by the process according to one of the preceding claims, characterized in that it consists of a purified animal or human tissue having been placed in contact with a fluid in the supercritical state, characterized in that it has a lipidic residue less than 2 % and a strength equivalent to or greater than that of natural bone.

16. Biomaterial according to claim 15, characterized in that it has a compression resistance of the order of 10 to 20 MPa.

## Patentansprüche

1. Verfahren zur Behandlung von tierischen oder menschlichen Knochengeweben für den Erhalt eines Biomaterials, das in einen Menschen implantiert und mechanisch beansprucht werden kann, wobei das Knochengewebe von der gesamten es umgebenden organischen Materie mechanisch gereinigt und die organische Materie aus dem knochengewebe extrahiert wird, dadurch gekennzeichnet, daß es wenigstens einen Schritt beinhaltet, in dem ein Fluid im überkritischen Zustand in die Gesamtheit des Knochengewebes eindringen gelassen wird.

2. Verfahren nach Anspruch 1, dadurch gekennzeichnet, daß das Knochengewebe in einem überkritischen Fluidstrom plaziert wird, der es durchfließt.

3. Verfahren nach einem der vorherigen Ansprüche, dadurch gekennzeichnet, daß der genannte Schritt ein Schritt ist, in dem die in dem Gewebe vorhandene, im wesentlichen lipidhaltige organische Materie extrahiert und in dem Fluid im überkritischen Zustand aufgelöst wird.

4. Verfahren nach einem der vorherigen Ansprüche, dadurch gekennzeichnet, daß es die folgenden Schritte umfaßt:
a) mechanisches Reinigen des Knochengewebes von der gesamten es umgebenden organischen Materie,
b) Zerschneiden dieses Knochengewebes nach einer vorbestimmten Form,
c) Reinigen des Knochengewebes, um daraus die für eine gute Neuimplantation schädlichen Bestandteile zu extrahieren, wobei in die Gesamtheit des Knochengewebes ein Fluid im überkritischen Zustand eindringen gelassen wird, das in der Lage ist, die in dem Gewebe vorhandene, im wesentlichen lipidhaltige organische Materie aufzulösen und zu extrahieren,
d) Waschen, Dehydrieren und Sterilisieren des auf diese Weise gereinigten Knochengewebes.

5. Verfahren nach Anspruch 4, dadurch gekennzeichnet, daß der Reinigungsschritt c) außerdem die chemische und/oder enzymatische Behandlung des knochengewebes beinhaltet, um bestimmte Proteinrückstände daraus zu extrahieren.

6. Verfahren nach einem der vorhergehenden Ansprüche, dadurch gekennzeichnet, daß das verwendete Fluid im überkritischen Zustand Kohlendioxid CO₂ ist.

7. Verfahren nach Anspruch 6, dadurch gekennzeichnet, daß das Kohlendioxid CO₂ im überkritischen Fluidzustand eine Temperatur in der Größenordnung von 31° bis 60°C aufweist und mit einem Druck in der Größenordnung von 1,5 x 10⁷ bis 4 x 10⁷ Pa beaufschlagt wird.

8. Verfahren nach einem der vorherigen Ansprüche, dadurch gekennzeichnet, daß das Fluid im überkritischen Zustand bestimmte aufgelöste Fluide enthält, wobei diese bestimmten Fluide zum Extrahieren von Proteinen oder anderen bestimmten Substanzen aus dem Knochengewebe verwendet werden können.

9. Verfahren nach einem der Ansprüche 1 bis 8, dadurch gekennzeichnet, daß bestimmte Fluide zum Extrahieren von bestimmten Proteinen aus dem Knochengewebe in einem Waschschritt verwendet werden, der nach dem Schritt durchgeführt wird, in dem das im überkritischen Zustand befindliche Fluid verwendet wird.

10. Verfahren nach einem der vorherigen Ansprüche, dadurch gekennzeichnet, daß das Knochengewebe durch Aluminiumoxidsandstrahlen mechanisch gereinigt wird.

11. Verfahren nach einem der vorherigen Ansprüche, dadurch gekennzeichnet, daß das Knochengewebe mit einem Hochdruckwasserstrahl zerschnitten wird.

12. Verfahren nach einem der vorherigen Ansprüche, dadurch gekennzeichnet, daß das Knochengewebe nach seiner Behandlung mit dem Fluid im überkritischen Zustand gewaschen wird, indem es durch mehrere aufeinanderfolgende Bäder mit destilliertem Wasser mit einer Temperatur zwischen 20° und 60°C geleitet wird.

13. Verfahren nach Anspruch 12, dadurch gekennzeichnet, daß das Knochengewebe nach dem Waschen dehydriert und desinfiziert wird, indem es durch mehrere aufeinanderfolgende Ethylalkoholbäder mit zunehmender Konzentration geleitet wird, worauf es in einem belüfteten Wärmeschrank mit einer Temperatur zwischen 31° und 60°C getrocknet wird.

14. Verfahren nach Anspruch 13, dadurch gekennzeichnet, daß das Knochengewebe nach der Dehydration nach dem Verpacken durch Bestrahlung sterilisiert wird.

15. Biomaterial, das in einen Menschen implantiert und gemäß dem Verfahren nach einem der vorherigen Ansprüche erhalten werden kann, dadurch gekennzeichnet, daß es aus tierischem oder menschlichem Knochengewebe besteht und mit einem Fluid im überkritischen Zustand in Kontakt gebracht wird, dadurch gekennzeichnet, daß es einen Lipidrückstand von weniger als 2% und einen Äquivalentwiderstand hat, der größer ist als der des natürlichen Knochens.

16. Biomaterial nach Anspruch 15, dadurch gekennzeichnet, daß es einen Kompressionswiderstand in der Größenordnung von 10 bis 20 MPa aufweist.
